# DEMANDE DE BREVET EUROPEEN

(11) **EP 4 047 614 A1**
(43) Date de publication de la demande: **24.08.2022**
(21) Numéro de dépôt: 22152147.9
(22) Date de dépôt: 19.01.2022
(51) Int. Cl.: G16H 40/20, G06Q 50/22

(54) **GESTION DE LA DISTRIBUTION DES BOUTEILLES DE GAZ DANS UN ÉTABLISSEMENT HOSPITALIER**

(30) Priorité: 18.02.2021 FR 2101576
(71) Demandeur: L'Air Liquide, société anonyme pour l'Étude et l'Exploitation des procédés Georges Claude, 75007 Paris (FR)
(72) Inventeur: BELLINGERI, Federica, 1804 Zemst (BE); POPOVIC, Stéphane, 94250 Gentilly (FR); CAMPAGNAC, Adrien, 75007 Paris (FR); MAS, Adrien, 75007 Paris (FR)
(74) Mandataire: Air Liquide

(57) **Abrégé**

L'invention concerne une méthode de gestion des bouteilles de gaz (2) mobiles au sein d'un établissement hospitalier (1) comprenant des services de soins (3a-3d) entre lesquels transitent les bouteilles de gaz (2) et dans lesquels elles sont utilisées. Chaque bouteille de gaz (2) est équipée d'un robinet de distribution de gaz (4) à dispositif électronique (5) pour déterminer et fournir au moins une information de quantité de gaz, et comprenant en outre un module de communication sans fil configuré pour transférer l'information de quantité de gaz, via un protocole de communication sans fil, à des moyens de traitement de données (7), via une pluralité de récepteurs (6), agencés dans les services de soins (3a-3d) de l'établissement hospitalier (1). Les moyens de traitement de données (7) traitent cette information de quantité de gaz et pilotent un affichage sur un écran d'affichage (8). Installation de gestion de bouteilles associée.

## Description

L'invention concerne une méthode de gestion améliorée de la distribution des bouteilles de gaz, en particulier d'oxygène médicinal, qui sont mobiles au sein d'un établissement hospitalier, c'est-à-dire qui peuvent être transportées d'un endroit à l'autre au sein de cet établissement hospitalier, l'oxygène médicinal étant de l'oxygène répondant à la Pharmacopée et répondant à une autorisation de mise sur le marché (AMM).

Au sein d'un établissement hospitalier, i.e. hôpital, clinique ou analogue, l'oxygène médicinal peut être distribué par le réseau de canalisations de gaz du bâtiment ou être utilisé sous forme conditionné dans des récipients de gaz, couramment appelés bouteilles de gaz, mais aussi bonbonnes de gaz ou analogue.

Lorsque l'oxygène médicinal est conditionné en bouteilles de gaz, il est principalement utilisé pour assurer le transfert des patients sous oxygénothérapie au sein des établissements hospitaliers, c'est-à-dire d'un service clinique à un autre, ou pour réaliser des examens dans des services de radiologie ou d'exploration fonctionnelle. On parle alors de bouteilles de gaz « mobiles » car elles sont amenées à être déplacées au sein des établissements hospitaliers entre le moment où elles commencent à être utilisées et le moment où elles sont renvoyées, après utilisation, vers le centre de remplissage du fournisseur de gaz.

Le personnel soignant doit concilier deux objectifs pouvant être contradictoires liés aux bouteilles de gaz « mobiles », à savoir assurer la sécurité du patient transféré en maintenant la continuité de son oxygénothérapie jusqu'au bout de son transfert et son arrivée dans un service où une nouvelle bouteille d'oxygène mobile sera disponible pour remplacer celle qui aura été vidée pendant le transfert et, par ailleurs, se préoccuper d'une utilisation efficace des bouteilles d'oxygène mobiles mises à sa disposition, y compris celles partiellement vidées, pour éviter un gaspillage d'oxygène et donc maîtriser le coût des soins prodigués aux patients.

En effet, en fonction de sa contenance et/ou de la consommation de l'oxygène qu'elle contient, une même bouteille de gaz peut être utilisée plusieurs fois, e.g. pour procéder aux transferts successifs de plusieurs patients.

Autrement dit, le personnel médical doit à la fois laisser un minimum d'oxygène résiduel dans les bouteilles ayant été utilisées, avant de renvoyer les bouteilles vides ou quasiment vides au centre de conditionnement du fournisseur de gaz, afin de minimiser les pertes et par ailleurs éviter absolument tout interruption de fourniture d'oxygène aux patients pendant leurs transferts ou analogues, donc faire attention à ne pas utiliser une bouteille de gaz mobile ne contenant pas assez d'oxygène médicinal pour garantir un transfert du patient en toute sécurité.

Cette problématique se pose différemment d'un service de soins à un autre, en fonction notamment de la criticité des patients qui y sont pris en charge, de la variété de leur profil au sein d'un même service de soin, ainsi que la durée des transferts réalisés à partir d'un service donné, donc de la quantité d'oxygène consommée pendant chaque transfert.

Le volume résiduel en oxygène en-dessous duquel une bouteille d'oxygène mobile ne peut plus être utilisée pour transférer un patient en toute sécurité va donc varier d'un service à l'autre. A titre d'exemple, il sera plus élevé dans un service de réanimation prenant soin de patients en situation critique que dans un service de médecine générale où sont hospitalisés des patients stables nécessitant des volumes d'oxygène médicinal plus limités.

US-A-2019/0107253 propose une méthode de suivi des bouteilles de gaz au sein d'un hôpital, dans laquelle chaque bouteille de gaz est équipée d'un robinet de distribution de gaz comprenant un dispositif électronique incluant un module de communication sans fil configuré pour émettre des données, via un protocole de communication sans fil, vers des balises réceptrices, agencés dans l'hôpital qui les renvoient vers un centre de traitement de données. Le dispositif électronique est équipé d'un écran d'affichage donnant des informations relatives à la bouteille de gaz sur laquelle il est installé.

Toutefois, cette méthode ne permet pas de réaliser une gestion précise des flux et de l'état des bouteilles de gaz dans l'hôpital. En particulier, elle ne permet pas de connaître précisément le nombre de bouteilles de chaque catégorie, i.e. pleines, vides ou partiellement vides, dans chaque service hospitalier et donc ne permet pas de gérer efficacement les flux de bouteilles dans l'hôpital, en particulier d'un service à l'autre, pour notamment minimiser les pertes de gaz et améliorer les réapprovisionnements en gaz, c'est-à-dire les flux de bouteilles entre l'hôpital et le ou les fournisseurs de gaz, i.e. le(s) centre de conditionnement.

Par ailleurs, US-A-2019/0162617 propose une méthode de calcul de l'autonomie en gaz d'une bouteille de gaz, dans laquelle un téléphone multifonction (i.e. smartphone) ou analogue est utilisé pour prendre une image du cadran du manomètre d'une bouteille de gaz, lequel cadran comprend un marquage de type QR-Code ou analogue en regard duquel se déplace une aiguille rotative dont la position vis-à-vis du marquage reflète la pression dans la bouteille considérée. L'image est traitée par le téléphone afin d'en déduire une autonomie en gaz.

Cette méthode est fastidieuse et ne permet pas de gérer efficacement les flux et l'état des bouteilles de gaz dans un hôpital puisqu'elle requiert que le cadran du manomètre de chaque bouteille soit pris en photo par le téléphone. De plus, l'écran du téléphone affiche au mieux une autonomie de la bouteille considérée mais ne donne aucune une information relative à l'ensemble du parc présent à un endroit donné. Elle n'est donc pas adaptée à la gestion des bouteilles de gaz réparties entre différents services de soins d'un établissement hospitalier.

Un problème est dès lors de pouvoir opérer une gestion efficace et en toute sécurité du parc de bouteilles de gaz, en particulier celles d'oxygène médicinal, réparties dans les différents services de soins d'un établissement hospitalier de manière à pouvoir disposer en permanence non seulement d'un état des différentes bouteilles qui s'y trouvent (i.e. pleines, vides ou partiellement vides, c'est-à-dire en cours d'utilisation) mais aussi de leur répartition par service, et préférentiellement aussi par taille ou autre et ce, dans le but d'améliorer les flux de bouteilles et les réapprovisionnements en minimisant les pertes de gaz.

Une solution de l'invention concerne alors une méthode, i.e. un procédé, de gestion des bouteilles de gaz mobiles au sein d'un établissement hospitalier comprenant plusieurs services de soins entre lesquels transitent des bouteilles de gaz et dans lesquels lesdites bouteilles de gaz sont utilisées, dans laquelle :
a) chaque bouteille de gaz est équipée d'un robinet de distribution de gaz comprenant un dispositif électronique configuré pour déterminer et fournir au moins une information de quantité de gaz dans la bouteille de gaz considérée, et comprenant en outre un module de communication sans fil configuré pour transférer ladite au moins une information de quantité de gaz, via un protocole de communication sans fil, et
b) l'établissement hospitalier est équipé d'une pluralité de récepteurs agencés au moins dans les services de soins et configurés pour recevoir ladite au moins une information de quantité de gaz transmise par chaque bouteille de gaz présente dans lesdits services de soins et pour transmettre ladite au moins une information de quantité de gaz à des moyens de traitement de données.

Selon la méthode de l'invention, les moyens de traitement de données sont configurés pour :
- traiter ladite au moins une information de quantité de gaz (Q_{g}) transmise pour chaque bouteille de gaz, et en déduire si chaque bouteille de gaz est au moins une bouteille de gaz pleine ou une bouteille de gaz vide et/ou préférentiellement une bouteille en cours d'utilisation (i.e. partiellement vide), et
- commander sur au moins un écran d'affichage, un affichage pour un ou des services de soins, d'au moins :
   - le nombre de bouteilles de gaz pleines (#P) présentes dans le ou les services de soins considérés et
   - le nombre de bouteilles de gaz vides (#V) présentes dans le ou les services de soins considérés,
   et préférentiellement :
   - le nombre de bouteilles de gaz en cours d'utilisation (i.e. une bouteille qui n'est plus totalement pleine et pas encore vide, c'est-à-dire qui a déjà commencé à être utilisée pour fournir du gaz) présentes dans le ou les services de soins considérés et/ou
   - le nombre total de bouteilles de gaz présentes dans le ou les services de soins considérés.

Selon le mode de réalisation considéré, la méthode de l'invention peut comprendre l'une ou plusieurs des caractéristiques suivantes :
- les moyens de traitement de données sont configurés pour commander en outre un affichage, pour un ou des services de soins, du nombre de bouteilles de gaz en cours d'utilisation et du nombre total de bouteilles de gaz présentes dans le ou les services de soins considérés.
- les moyens de traitement de données sont configurés pour déterminer le nombre total de bouteilles dans au moins l'un des services de soins considérés, c'est-à-dire le stock de bouteilles présentes, à partir des nombres de bouteilles vides, pleines et en cours d'utilisation.
- le nombre total de bouteilles correspond à la somme des nombres de bouteilles vides, pleines et en cours d'utilisation (i.e. ni totalement vides, ni totalement pleines).
- les moyens de traitement de données sont configurés pour commander en outre un affichage d'une dotation de bouteilles pour l'un ou plusieurs desdits services de soins considérés, c'est-à-dire le stock de bouteilles allouées audit service.
- chaque bouteille de gaz est identifiée par une identification (ID) de bouteille spécifique, c'est-à-dire un identifiant unique et spécifique de chaque bouteille de gaz.
- l'identification (ID) spécifique de chaque bouteille est mémorisée au sein du dispositif électronique équipant le robinet de distribution de gaz.
- le dispositif électronique est configuré pour associer l'information de quantité de gaz relative (Q_{g}) à une bouteille de gaz donnée à l'identification (ID) de la bouteille de gaz considérée, en particulier un microprocesseur agencé dans ledit dispositif électronique.
- l'identification (ID) de chaque bouteille est un numéro de série.
- l'information de quantité de gaz et l'identification (ID) de la bouteille de gaz sont transmises associées l'une à l'autre.
- le dispositif électronique est configuré pour déterminer et fournir au moins une information de quantité de gaz (Q_{g}) choisie parmi une pression de gaz ou un volume de gaz.
- le module de communication sans fil est configuré pour transférer l'information de quantité de gaz correspondant à chaque bouteille de gaz associée à l'identification (ID) de chaque bouteille de gaz considérée.
- chaque récepteur, i.e. les récepteurs, est identifié par une identification de récepteur (ID_{R}), c'est-à-dire unique et/ou qui lui est propre.
- les récepteurs sont configurés pour transmettre aux moyens de traitement de données, au moins une information de quantité de gaz (Q_{g}) associée à une identification (ID) de bouteille et à au moins une identification du récepteur (ID_{R}) ayant reçu ladite au moins une information de quantité de gaz (Q_{g}) associée à une identification (ID) de bouteille.
- l'identification de récepteur (ID_{R}) spécifique de chaque récepteur est mémorisée au sein du récepteur considéré.
- les moyens de traitement de données sont configurés pour traiter ladite au moins une information de quantité de gaz (Q_{g}) transmise pour chaque bouteille de gaz associée à une identification (ID) de bouteille et l'identification de récepteur (ID_{R}) ayant recueilli ladite au moins une information de quantité de gaz (Q_{g}) de manière à attribuer (i.e. associer) ladite au moins une information de quantité de gaz (Q_{g}) associée à une identification (ID) de bouteille à au moins un service de soins du bâtiment hospitalier correspondant à ladite identification de récepteur (ID_{R}).
- les (i.e. chaque) récepteurs sont configurés pour transmettre des ensembles de données (Q_{g}, ID, ID_{R}) comprenant une information de quantité de gaz (Q_{g}), une identification (ID) de bouteille et une identification du récepteur (ID_{R}).
- les moyens de traitement de données sont configurés pour traiter les ensembles de données (Q_{g}, ID, ID_{R}) transmis par le ou les récepteurs.
- ladite au moins une identification du récepteur (ID_{R}) est associée à (i.e.appariée) et/ou correspond à au moins un service de soins du bâtiment hospitalier.
- chaque association de l'identification d'un récepteur (ID_{R}) à un service de soins, c'est-à-dire les correspondances entre les identifications de récepteur (ID_{R}) et les services de soins, est mémorisée au sein de moyens de mémorisation.
- les moyens de traitement de données sont configurés pour aller retrouver les associations entre identification d'un récepteur (ID_{R}) et service de soins correspondant ayant été mémorisées.
- l'information de quantité de gaz, l'identification de récepteur (ID_{R}) et/ou l'identification (ID) de la bouteille de gaz sont transmises associées sous forme d'une trame ou d'un codage informatique.
- la trame ou codage informatique comprend l'identification (ID) de la bouteille considérée, l'information de quantité de gaz et l'identification de récepteur (ID_{R}), et éventuellement une ou plusieurs autres informations additionnelles, en particulier les ensembles de données (Q_{g}, ID, ID_{R}).
- chaque service de soin comprend au moins un récepteur.
- les récepteurs forment un réseau de récepteurs au sein du bâtiment hospitalier.
- les récepteurs sont fixés aux murs ou aux plafonds des services de soins de l'établissement hospitalier.
- les récepteurs sont alimentés électriquement.
- les moyens de traitement de données sont agencés dans l'établissement hospitalier ou sont situés à distance de l'établissement hospitalier.
- les moyens de traitement de données comprennent un ou des microprocesseurs.
- les moyens de traitement de données comprennent un (ou des) ordinateur ou serveur informatique.
- les moyens de traitement de données sont configurés pour comparer ladite au moins une information de quantité de gaz (Q_{g}) transmise pour chaque bouteille de gaz comprise dans un même service de soin avec au moins une valeur-seuil haut (VS) et une valeur-seuil bas (VSB) associée au service de soin considéré et pour en déduire si chaque bouteille de gaz est :
   ▪ une bouteille de gaz pleine lorsque ladite au moins une information de quantité de gaz (Q_{g}) est supérieure ou égale à ladite valeur-seuil haut (VSH), i.e. Q_{g} ≥ VSH, ou
   ▪ une bouteille de gaz vide lorsque ladite au moins une information de quantité de gaz (Q_{g}) est inférieure à ladite valeur-seuil bas (VSB), i.e. Q_{g} < VSB, avec VSH > VSB, ou
   ▪ une bouteille de gaz en cours d'utilisation lorsque ladite au moins une information de quantité de gaz (Q_{g}) est comprise entre les valeur-seuil haut (VSH) et valeur-seuil bas (VSB), i.e. VSB < Q_{g} < VSH.
- les valeur-seuil haut (VSH) et valeur-seuil bas (VSB) sont des valeurs de pression (par exemple exprimées en bar) ou de volume de gaz (par exemple exprimées en litre).
- les valeur-seuil haut (VSH) et valeur-seuil bas (VSB) sont mémorisées par le dispositif électronique.
- les valeur-seuil haut (VSH) et valeur-seuil bas (VSB) sont paramétrables, c'est-à-dire peuvent être ajustées ou modifiées par l'utilisateur, par exemple paramétrables par service de soins, notamment pour prendre en compte des spécificités liées aux différents service de soins.
- la valeur-seuil haut (VSH) est supérieure ou égale à 150 bar environ.
- la valeur-seuil bas (VSB) est inférieure ou égale à 50 bar environ.
- le dispositif électronique comprend des moyens de mémorisation pour mémoriser/enregistrer des données, des valeurs ou autres informations.
- les moyens de mémorisation comprennent au moins une mémoire informatique, par exemple une mémoire flash ou toute autre mémoire adaptée.
- les moyens de traitement de données sont configurés pour déterminer le nombre total de bouteilles de gaz présentes dans chaque service de soins.
- les moyens de traitement de données du dispositif électronique comprennent un ou plusieurs microprocesseur(s), en particulier un microcontrôleur.
- les moyens de traitement de données du dispositif électronique comprennent un ou plusieurs microprocesseur(s) mettant en oeuvre un ou des algorithmes.
- l'écran d'affichage est un écran d'ordinateur, de tablette numérique ou de téléphone multifonction.
- les services de soins sont choisis parmi les urgences, les services de traumatologie, les services pédiatriques, les services de chirurgie, les services de soins intensifs ou tout autre service hospitalier dans lequel se pratique des soins aux patients.
- l'établissement hospitalier est un hôpital, une clinique ou analogue.
- les moyens de traitement de données sont en outre configurés pour associer une bouteille de gaz donnée à un service de soins donné, lorsque la bouteille de gaz considérée est détectée par un (ou le) récepteur agencé dans le service de soins considéré, pendant une durée (dt) prédéfinie, c'est-à-dire après un seuil de temps paramétrable par l'utilisateur, par exemple une durée de 0 à plusieurs dizaines de minutes, i.e. pouvant atteindre 1 à plusieurs heures. Dans le cas où la durée est fixée à 0, la bouteille de gaz considérée est associée à un service de soins donné, c'est-à-dire considérée comme en faisant partie, dès qu'elle y est détectée, c'est-à-dire sans phase de latence.
- les moyens de traitement de données sont configurés pour commander l'affichage sur ledit au moins un écran d'affichage, pour au moins un service de soins, au moins le nombre de bouteilles de gaz pleines et/ou de bouteilles de gaz vides présentes dans ledit au moins un service de soins considéré, de préférence on affiche aussi le nombre de bouteilles de gaz en cours d'utilisation, c'est-à-dire qui ne sont ni pleines, ni vides mais contenant une quantité de gaz intermédiaire.
- les bouteilles de gaz sont affichés en étant réparties par taille ou contenance, et/ou en fonction du gaz qu'elles contiennent.
- les bouteilles contiennent un gaz médical choisi parmi l'oxygène ou l'air médicinal, ou un mélange NO/N₂, O₂/N₂O, He/O₂, Ar/O₂, Ar/N₂/O₂ Xe/O₂ ou tout autre gaz médicinal.
- les bouteilles contiennent préférentiellement de l'oxygène médicinal.
- les bouteilles ont des volumes internes compris entre 0,5 et 20 Litres (équivalent en eau) environ.
- le dispositif électronique comprend au moins un capteur, en particulier un capteur de pression.
- le dispositif électronique comprend au moins des moyens de contrôle à (au moins un) microprocesseur, de préférence le (les) microprocesseur est porté par une carte électronique.
- le module de communication sans fil est agencé dans le dispositif électronique.
- le dispositif électronique est alimenté en courant électrique par une source de courant électrique afin d'alimenter électriquement ses composants nécessitant du courant électrique pour fonctionner, notamment le ou les capteurs, moyens de contrôle à microprocesseur, module de communication sans fil....
- la source de courant électrique comprend une (ou des) batterie ou pile, de préférence rechargeable.
- la bouteille comprend un capotage de protection agencé autour du RDI afin de le protéger contre les chocs ou autres agressions, en particulier un capotage de protection en polymère, en métal ou tout autre matériau.
- le dispositif électronique est logé dans une ouverture pratiquée dans le capotage de protection.
- la source de courant électrique est agencée dans un logement du capotage de protection et raccordée électrique au dispositif électronique.
- le dispositif électronique est solidaire du RDI, c'est-à-dire porté par le RDI.
- le dispositif électronique comprend un boitier externe contenant tout ou partie de ses composants.
- les bouteilles de gaz pleines sont stockées dans les services de soins avant d'être utilisées.
- les bouteilles de gaz vides sont stockées, au moins temporairement, dans les services de soins avant d'être évacuées.
- les moyens de traitement de données sont en outre configurés pour déterminer et commander l'affichage sur ledit au moins un écran d'affichage du nombre de bouteilles de gaz pleines à livrer à un premier service de soins et/ou du nombre de bouteilles de gaz dudit premier service de soins à affecter à un second service de soins, en fonction du nombre de bouteilles vides et/ou pleines, du nombre total de bouteilles et de la dotation desdits premier et/ou second service de soins.

L'invention va maintenant être mieux comprise grâce à la description détaillée suivante, faite à titre illustratif mais non limitatif, en référence aux figures annexées parmi lesquelles :
Fig. 1 schématise un mode de réalisation de la méthode de gestion des bouteilles de gaz mobiles au sein d'un établissement hospitalier selon l'invention ; et
Fig. 2 schématise l'écran d'affichage d'un ordinateur affichant le nombre de bouteilles de gaz conformément à la méthode de l'invention.

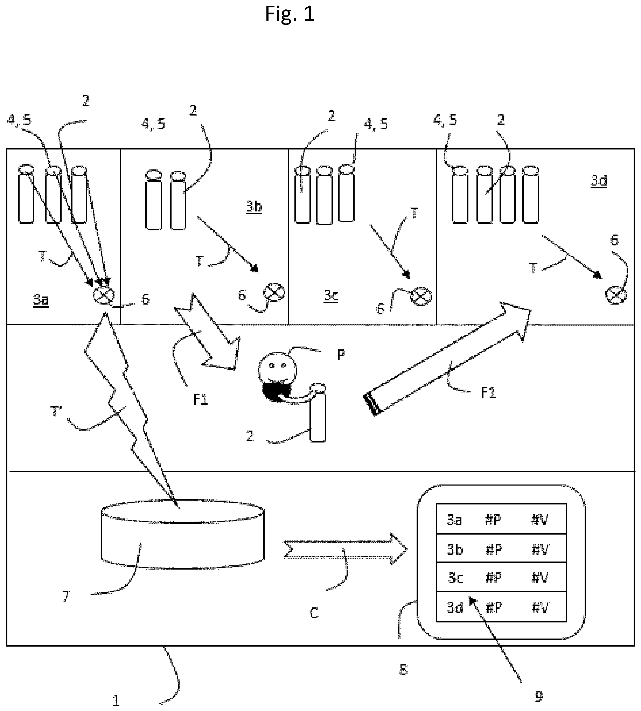

Fig. 1 schématise un mode de réalisation de la méthode de gestion des bouteilles de gaz 2, typiquement des bouteilles d'oxygène médicinal, mobiles au sein d'un établissement hospitalier 1, à savoir un bâtiment où des soins hospitaliers sont pratiqués sur des patients, tel qu'un hôpital, une clinique ou analogue.

L'établissement hospitalier 1 comprend plusieurs services de soins 3a-3d, par exemple des services de pédiatrie, d'urgences, de traumatologie, de chirurgie.... ou autres, entre lesquels transitent (flèches F1 et F2) les bouteilles de gaz 2 pendant le transfert des patients P d'un service à un autre. Chaque service de soins 3a-3d comprend une ou plusieurs pièces ou analogues.

Les bouteilles de gaz 2 sont donc stockées et/ou utilisées dans les différents services de soins 3a-3d, voire que pendant les phases de transfert interservices.

Ainsi, en Fig. 1, on a schématisé le transfert (flèches F1 et F2) par le personnel soignant, par exemple un brancardier ou un infirmier, d'un patient P alimenté en oxygène par une bouteille d'oxygène médicinal 2, via un masque respiratoire et un tuyau flexible, depuis un service de soins 3a à un autre service de soins 3d, par exemple depuis un service d'urgences vers un service de traumatologie.

Chaque bouteille de gaz 2, typiquement d'oxygène médicinal, est équipée d'un robinet de distribution de gaz 4, de préférence un robinet à détendeur intégré (RDI), comprenant un dispositif électronique 5 et éventuellement un capotage de protection agencé autour du robinet de distribution de gaz 4 et/ou du dispositif électronique 5 pour les protéger contre les chocs ou autres.

Le dispositif électronique 5 est par exemple une jauge ou un manomètre digital permettant de mesurer et fournir une information de quantité de gaz (Q_{g}) dans la bouteille de gaz 2 sur laquelle il est installé, typiquement la pression ou le volume de gaz résiduel, par exemple au moyen d'un capteur de pression.

Les bouteilles de gaz 2 sont identifiées par une identification (ID) de bouteille spécifique. Cette identification (ID) de bouteille est mémorisée par le dispositif électronique 5 équipant le RDI 4 équipant chaque bouteille 2. L'identification (ID) de chaque bouteille est de préférence un numéro de série.

L'information de quantité de gaz (Q_{g}) relative à une bouteille de gaz donnée, par exemple une pression ou un volume de gaz, est associée à l'identification (ID) de la bouteille de gaz considérée de manière à pouvoir savoir à quelle bouteille correspond chaque information de quantité de gaz captée. On obtient alors une trame ou un codage informatique qui peut être transmis et qui contient au moins une identification (ID) de bouteille de gaz associée à une quantité de gaz (i.e. pression ou volume) déterminée et optionnellement une ou plusieurs autres infirmations.

Le dispositif électronique 5 inclut aussi un module de communication sans fil configuré pour transférer (T) l'information de quantité de gaz associée l'identification (ID) de bouteille, via un protocole de communication sans fil, par exemple en mode Bluetooth, par exemple en Bluetooth Basse Energie (BLE), ou un autre protocole adapté, par exemple un protocole de type UHF, infra-rouge (IR), Lora, Sigfox ou autre. Le module de communication sans fil comprend par exemple un microcontrôleur et une antenne émettrice.

Une batterie ou analogue alimente en courant électrique le dispositif électronique 5 de chaque bouteille 2, en particulier ses composants internes qui requièrent du courant électrique pour fonctionner. La batterie peut être logée dans un compartiment à batterie du capotage de protection protégeant le RDI 4 de chaque bouteille 2 de gaz.

Le dispositif électronique 5 de chaque bouteille 2 communique avec des récepteurs 6, c'est-à-dire des balises ou analogues, agencés au moins dans les différents services de soins 3a-3d de l'établissement hospitalier 1, c'est-à-dire dans les différentes pièces, salles, chambres ou lieux dans lesquelles les bouteilles de gaz 2 sont susceptibles d'être stockées et/ou utilisées.

Les récepteurs 6 comprennent typiquement des moyens émetteurs/récepteurs, de préférence sans fil, permettant de recevoir les informations, i.e. mesures, données, signaux ou autres, transmises par les dispositifs électroniques 5 équipant les différentes bouteilles 2 associées à l'ID de chaque bouteille, et pour ensuite les transmettre (T') à des moyens de traitement de données 7, de préférence une (ou des) unité à microprocesseur(s), tel qu'un ordinateur ou un serveur informatique distant ou agencé dans l'établissement hospitalier 1 ou à proximité.

Plus précisément, chaque récepteur 6 est lui-même identifié par une identification de récepteur ID_{R} unique, c'est-à-dire qui lui est propre. Cette identification de récepteur ID_{R} unique est mémorisée par le récepteur 6 considéré. Chaque identification de récepteur (ID_{R}) correspond en fait à un service de soins du bâtiment hospitalier donné.

Lorsqu'un récepteur 6 reçoit une information de quantité de gaz (Q_{g}) associée à une ID de bouteille, il retransmet ensuite aux moyens de traitement de données, cette information de quantité de gaz (Q_{g}) et cette ID de bouteille associées à sa propre identification de récepteur (ID_{R}), c'est-à-dire des ensembles de données (Q_{g}, ID, ID_{R}).

Par exemple, l'information de quantité de gaz, l'identification de récepteur (ID_{R}) et l'identification (ID) de la bouteille de gaz, c'est-à-dire les ensembles de données (Q_{g}, ID, ID_{R}), sont transmises associées sous forme d'une trame ou d'un codage informatique, qui peut en outre éventuellement comprendre une ou plusieurs autres informations additionnelles.

Les récepteurs 6 peuvent communiquer avec les moyens de traitement de données 7 en Bluetooth ou par un réseau de type internet, intranet ou analogue, ou via un autre protocole, tel que Lora, Sigfox ou un réseau cellulaire par exemple.

Autrement dit, les bouteilles d'oxygène médicinal 2 sont toutes équipées d'un dispositif électronique 5 de type jauge digitale ou similaire permettant de connaître à tout moment la valeur exacte de la pression et du volume résiduel en oxygène de chaque bouteilles d'oxygène médicinal 2 et par ailleurs mémorisant l'ID de chaque bouteille sur lequel il est monté.

On prévoit sur chaque bouteille 2, un module de communication sans fil, c'est-à-dire un module communiquant, permettant de transférer l'information de quantité de gaz, en particulier de pression et volume, voire d'autres informations, associées à l'ID bouteille de la bouteille 2 considérée par un protocole de communication sans fil, de sorte que ces informations puissent être captées ensuite par un réseau de récepteurs 6 faisant transiter les données ou informations vers des moyens de traitement de données 7 incluant notamment une base de donnée centralisée.

Préférentiellement, le module de communication sans fil est intégré au dispositif électronique 5 de type jauge digitale ou similaire équipant chaque bouteille de gaz 2.

De plus, selon l'invention, les moyens de traitement de données 7 sont configurés pour traiter l'information de quantité de gaz (Q_{g}) transmise pour toutes les bouteilles de gaz 2 présentes dans les différents services de soins 3a-3d de l'établissement hospitalier 1, laquelle est associée à l'ID de chacune des bouteilles et à l'identification d'un récepteur (ID_{R}), donc d'un service de soins donné, et en déduire ensuite si chaque bouteille de gaz 2 est une bouteille de gaz pleine ou une bouteille de gaz vide, ou préférentiellement encore une bouteille en cours d'utilisation, c'est-à-dire contenant une quantité de gaz intermédiaire, et préférentiellement en déduire par ailleurs le nombre total de bouteilles 2 présentes dans un ou plusieurs services de soins 3a-3d, de préférence dans chaque service de soins 3a-3d.

En particulier, les moyens de traitement de données sont configurés pour traiter l'information de quantité de gaz (Q_{g}) associée à l'ID de chaque bouteille de gaz 2, et l'identification de récepteur (ID_{R}) ayant recueilli ladite information de quantité de gaz (Q_{g}) de manière à attribuer ou associer ladite information de quantité de gaz (Q_{g}) à un service de soins 3a-3d du bâtiment hospitalier correspondant à l'identification de récepteur (ID_{R}).

Les correspondances entre les identifications (ID_{R}) des récepteurs 6 et les différents services de soins 3a-3d qui leur sont associés, sont mémorisées au sein de moyens de mémorisation, telle une mémoire informatique indépendante ou directement compris dans les moyens de traitement de données 7. Dans tous les cas, les moyens de traitement de données 7 vont aller retrouver les associations entre identification d'un récepteur (ID_{R}) et service de soins 3a-3d correspondant ayant été mémorisées.

En outre, les moyens de traitement de données 7 sont configurés pour commander sur un (ou des) écran d'affichage 8, un affichage 9 pour les différents services de soins 3a-3d, tout ou partie des nombres de bouteilles 2 ayant été déterminés, en particulier le nombre de bouteilles de gaz 2 pleines (#P) et/ou le nombre de bouteilles de gaz 2 vides (#V) et/ou le nombre de bouteilles en cours d'utilisation et/ou le nombre total de bouteilles présentes dans le ou les services de soins 3a-3d considérés.

Ainsi, un utilisateur, tel du personnel soignant, ayant besoin d'utiliser une bouteille de gaz 2 pour assurer un transfert d'un patient depuis ce service de soins 3a-3d vers un autre service de soins 3a-3d, peut immédiatement savoir si les bouteilles de gaz 2 présentes dans le lieu où il se trouve sont des bouteilles de gaz 2 pleines (#P), des bouteilles de gaz 2 vides (#V), ou des bouteilles en cours d'utilisation, et connaître le nombre de bouteilles de chaque catégorie, lesquelles peuvent aussi être affichées en étant réparties par tailles lorsque les bouteilles ne sont pas toutes de la même taille, par exemple des bouteilles de 2L, 5L et 11L (équiv. en eau), ou encore réparties en fonction de la nature du gaz qu'elles renferment, par exemple oxygène, air...

L'ensemble de ces informations est affiché sur un écran d'affichage comme expliqué ci-après.

Grâce à l'invention, on peut connaître en temps réel, le nombre total de bouteilles 2 présentes dans chaque service de soins 3a-3d, et discriminer, selon les désirs de la personne qui a besoin de ces informations, entre les bouteilles pleines (#P), les bouteilles vides (#V), et/ou les bouteilles en cours d'utilisation, et même, si besoin est, les obtenir classées par taille ou autre et/ou par type de gaz qu'elles contiennent (i.e. O₂, air...).

Pour ce faire, les moyens de traitement de données 7 sont configurés pour comparer l'information de quantité de gaz (Q_{g}) provenant de toutes bouteilles de gaz 2 comprises dans un même service de soin (3a-3d) avec des valeurs-seuils (VS) prédéfinies, en particulier une valeur-seuil bas (VSB) et une valeur-seuil haut (VSH), avec VSH > VSB, par exemple des pressions basse et haute ou des volumes bas et haut de référence, lesquelles valeurs-seuils sont préenregistrées, i.e. mémorisés par des moyens de mémorisation, et préférentiellement associées au(x) service(s) de soins 3a-3d considéré(s).

Autrement dit, on peut définir des valeurs-seuils spécifiques de chaque service de soins ou alors fixer de mêmes valeurs-seuils pour plusieurs ou tous les services.

Les moyens de traitement de données 7 déduisent ensuite de ces comparaisons si les différentes bouteilles de gaz 2 sont des bouteilles de gaz pleines ou vides, ou encore des bouteilles en cours d'utilisation, c'est-à-dire des bouteilles qui ne sont plus totalement pleines car une partie du gaz a été utilisée mais qui ne sont pas encore vides.

Une bouteille 2 est considérée comme une bouteille pleine pour le service hospitalier où elle se trouve, lorsque l'information de quantité de gaz (Q_{g}) pour cette bouteille 2 est supérieure ou égale à la valeur-seuil haut (VSH) préenregistrée pour le service concerné, c'est-à-dire Q_{g} ≥ VSH, par exemple une pression haute ou un volume haut.

A l'inverse, une bouteille 2 est considérée comme une bouteille vide pour le service hospitalier où elle se trouve, lorsque l'information de quantité de gaz (Q_{g}) pour cette bouteille 2 est inférieure à une valeur-seuil bas (VSB), i.e. Q_{g} < VSB, la valeur-seuil bas (VSB) étant bien entendu très inférieure à la valeur de valeur-seuil haut (VSH).

Par exemple, la valeur-seuil haut (VSH) de pression peut être fixée à 150 bar, voire à 180 bar, et la valeur-seuil bas (VSB) de pression peut être fixée à 50 bar, voire à 30 bar ; ou d'autres valeurs.

De façon analogue, exprimé en litres, la valeur-seuil haut (VSH) peut être par exemple fixée à environ 750 L et la valeur-seuil bas (VSB) peut être par exemple de l'ordre de 250 L pour une bouteille de 5L de volume (équiv. en eau) qui contient au maximum 1000L d'oxygène, c'est-à-dire dans ce cas, environ 75% et 25% du volume maximal, respectivement. Bien entendu, on peut choisir d'autres valeurs pour les deux seuils.

De là, une bouteille est considérée en cours d'utilisation lorsque l'information de quantité de gaz (Q_{g}) pour cette bouteille 2 est comprise entre les valeurs-seuils haut (VSH) et bas (VSB), ce qui correspond à une bouteille partiellement pleine.

En procédant ainsi, bouteille par bouteille 2 et service par service 3a-3d, les moyens de traitement de données 7 peuvent déterminer et comptabiliser les bouteilles de gaz vides, pleines et en cours d'utilisation présentes, à un instant donné, dans les différents services de soins 3a-3d de l'établissement 1, et donc aussi suivre leurs déplacements entre ces différents services de soins 3a-3d, résultant des transferts de patients P entre services.

De plus, connaître les bouteilles de gaz vides, pleines et en cours d'utilisation présentes, à un instant donné, dans les différents services de soins 3a-3d de l'établissement 1 permet en outre de connaître le nombre total de bouteilles de gaz 2 présentes dans les différents services à cet instant donné.

Une vue globale de la situation peut alors être affichée 9 sur un (ou des) écran d'affichage 8, par exemple l'écran d'un ordinateur, d'une tablette tactile ou d'un téléphone multifonction (i.e. smartphone).

Chaque service de soins 3a-3d qui dispose d'un écran d'affichage 8, peut être alors informé en temps réel des bouteilles 2 vides, pleines et en cours d'utilisation qui s'y trouvent. Comme déjà dit, les bouteilles 2 peuvent être affichées en étant par ailleurs réparties par taille et/ou par type de gaz qu'elles contiennent.

On peut aussi afficher la dotation en bouteilles 2 de chaque service afin de pouvoir visualiser et/ou détecter tout nombre excédentaire ou, à l'inverse, déficitaire de bouteilles 2 dans un (ou plusieurs) service donné et pouvoir y remédier en y ajustant le nombre de bouteilles 2 qui s'y trouvent, notamment lors des réapprovisionnements, c'est-à-dire l'enlèvement des bouteilles vides et leur remplacement par des bouteilles pleines.

Toutes ces informations peuvent être par ailleurs réunies sur un seul et même écran centralisant tous les suivis de bouteilles au sein de l'établissement hospitalier. Ceci facilite énormément la gestion globale du parc de bouteilles de gaz au sein de l'hôpital ou analogue.

Préférentiellement, chaque bouteille de gaz 2 est associée au stock du service 3a-3d dans lequel elle se trouve, c'est-à-dire à la totalité des bouteilles 2 qui se trouvent dans ce service, grâce à sa localisation par le récepteur 6 présent dans ce service clinique 3a-3d et faisant transiter les données vers les moyens de traitement de données 7 qui les traitent informatiquement.

Afin d'éviter qu'une bouteille 2 attribuée au stock d'un service clinique donné ne change plusieurs fois d'affectation au fil du transfert d'un patient P dans l'hôpital, puisque les informations transmises par la bouteille 2 considérée peuvent être captées successivement par les récepteurs 6 des services cliniques 3a-3d traversés par le patient P pendant son transfert interservices, le service de soins 3a-3d associé à une bouteille 2 donné n'est modifié qu'en cas de détection de la bouteille considérée 2 par un même récepteur 6, pendant une durée minimale définie par un seuil de temps paramétrable par l'utilisateur, de préférence une durée minimale mémorisée. Dans certains cas, cette durée est nulle (i.e. 0), c'est-à-dire que les bouteilles 2 sont immédiatement affectées au stock d'un service dès qu'elles y sont détectées. Dans d'autres cas, elles n'y sont affectées qu'après une durée de présence de plusieurs minutes, dizaines de minutes, voire même d'une ou plusieurs heures.

L'information du contenu résiduel en gaz, e.g. en oxygène, (i.e. pression ou volume résiduel) de chaque bouteille 2 est analysée par les moyens de traitement de données 7 en étant comparée à une ou des valeurs-seuils VS pour déterminer si chaque bouteille 2 est pleine, vide ou en cours d'utilisation (i.e. partiellement vide). Chaque valeur-seuil peut être définie de manière différenciée pour chaque service de soins 3a-3d de l'hôpital 1, de sorte d'être adaptée le mieux possible aux besoins et contraintes de chaque service de soins 3a-3d.

En particulier, les bouteilles 2 "vides" peuvent être ainsi détectées et identifiées pour chaque service. Toutefois, lorsqu'une bouteille 2 est considérée comme "vide" dans le service dans lequel elle se trouve mais que son contenu est supérieur à la valeur-seuil bas VSB, c'est-à-dire la valeur minimale, définie pour un autre service de soins moins critique, les moyens de traitement de données 7 sont configurés pour indiquer alors à l'utilisateur, une recommandation de transfert de la bouteille 2 du service d'origine vers le service dans lequel son contenu résiduel pourra être utilisé pour transférer un nouveau patient en toute sécurité.

A l'inverse, lorsqu'une bouteille 2 est considérée comme "vide" pour le service dans lequel elle se trouve, et qu'aucun autre service n'a un seuil minimal inférieur à son contenu résiduel, les moyens de traitement de données 7 sont configurés pour indiquer à l'utilisateur de remplacer cette bouteille 2 vide par une bouteille pleine

L'utilisateur a donc accès à l'information qu'une bouteille 2 est vide et doit être remplacée quasiment en temps réel, ce qui permet d'éviter tout risque de pénurie de bouteilles 2 utilisables dans les services de soins.

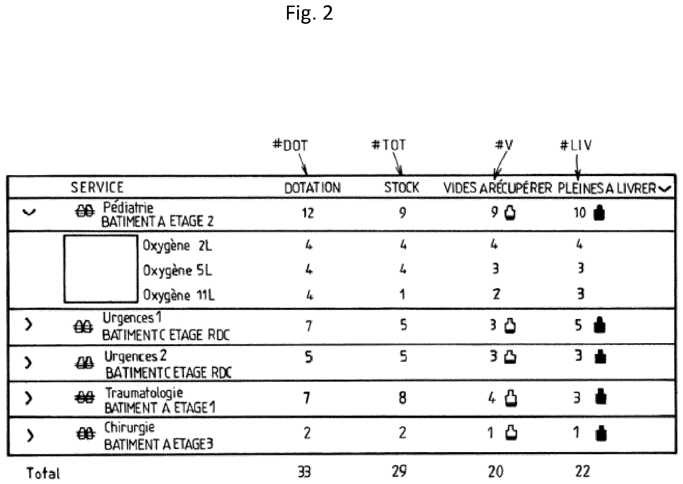

L'ensemble des informations et recommandations est affichée selon une répartition par service de soins et par exemple de tailles de bouteilles 2, comme illustré en Fig. 2.

Ainsi, Fig. 2 représente l'écran d'affichage 8 d'un ordinateur ou analogue, sur lequel les moyens de traitement de données 7 commandent un affichage 9 pour plusieurs services de soins 3a-3d, de nombres de bouteilles d'oxygène 2 qui s'y trouvent, à savoir ici deux services d'urgences et les services de traumatologie, de pédiatrie et de chirurgie de l'hôpital.

Plus précisément, comme on le voit, sont affichés pour chacun de ces services, le nombre total de bouteilles de gaz en stock (#TOT) et le nombre de bouteilles de gaz vides (#V) qui s'y trouvent.

Sont affichés aussi la dotation (#DOT) en bouteilles de chaque service, c'est-à-dire le nombre « cible » de bouteilles qui lui est attribué, et le nombre de bouteilles nouvelles devant être livrées (#LIV) à chaque services compte tenu de sa dotation, du stock qui s'y trouve et du nombre de bouteilles vides dans ce stock.

Le nombre total de bouteilles de gaz en stock (#TOT) correspond à la somme du nombre de bouteilles vides, de bouteilles pleines et de bouteilles en cours d'utilisation. Il est à noter que le nombre de bouteilles pleines (#P) et/ou en cours d'utilisation pourrait aussi être affiché.

Par ailleurs, comme on le voit, pour le service de pédiatrie, on affiche les bouteilles d'oxygène classées par taille ou contenance, à savoir ici des bouteilles d'oxygène de 2L, 5L et 11L de manière connaître précisément le nombre de bouteilles de chaque catégorie (i.e. taille) présentes et/ou devant être réapprovisionné (#LIV), ainsi que le nombre de bouteilles vides (#V) devant être enlevées et renvoyées dans le centre de conditionnement du fournisseur de gaz afin de les remplir avec du gaz frais. Là encore, on pourrait afficher aussi le nombre de bouteilles pleines (#P) et/ou en cours d'utilisation par catégories.

De même, on pourrait aussi afficher les bouteilles 2 présentes dans toute ou partie de ces services qui contiennent un gaz autre que l'oxygène, par exemple des bouteilles d'air médicinal ou d'un autre gaz médicinal.

Toutes ces informations permettent d'assurer un meilleur suivi et contrôle des flux de bouteilles dans l'hôpital, service par service.

Ainsi, pour le service de pédiatrie, on comprend que 9 bouteilles vides sont à récupérer, i.e. à enlever, et à renvoyer chez le fournisseur de gaz, comprenant 4 bouteilles de 2L, 3 bouteilles de 5L et 2 bouteilles de 11L, et que 10 bouteilles pleines doivent être réapprovisionnées pour être conforme à la dotation de ce service, à savoir 4 bouteilles de 2L, 3 bouteilles de 5L et 3 bouteilles de 11L

De même, on voit que le service de traumatologie comprend un nombre excessif de bouteilles (i.e. 8 bouteilles), c'est-à-dire supérieur à la dotation de ce service (i.e. 7 bouteilles). Dès lors, les 4 bouteilles vides présentes dans ce service seront remplacées par uniquement 3 bouteilles pleines pour, là encore, être conforme à la dotation de ce service.

Autrement dit, grâce à l'invention, on peut prendre en compte facilement l'écart entre la dotation cible d'un service et l'état réel du stock de bouteilles dans ce service et corriger tout écart éventuel en attribuant à ce service le nombre exact de bouteilles auquel il a droit, tout en prenant en compte les fluctuations du nombre de bouteilles au sein de ce service au fil du temps, notamment des allers-retours de patients dans ce service. Cela permet de garantir dans le temps, une affectation efficace du stock de bouteilles de l'hôpital en fonction des besoins de chaque service.

De plus, cela permet aussi d'opérer une gestion intelligente des bouteilles entre les services de l'hôpital, par exemple en opérant des transferts de bouteilles d'un service à un autre afin de se conformer aux dotations des services concernés, par exemple d'organiser un transfert d'un service excédant sa dotation du fait d'un nombre de bouteille stockées qui s'y trouvent vers un autre service dans lequel la quantité de bouteilles qui s'y trouvent est insuffisante, c'est-à-dire inférieure à sa dotation.

Les recommandations de transfert peuvent être faites aussi en tenant compte de seuils de bouteilles vides (VSB) différents. Ainsi un premier service, tels les soins intensifs par exemple, peut avoir un seuil VSB1 supérieur au seuil VSB2 d'un second service moins critique, par exemple la pédiatrie. Dans ce cas, une bouteille affectée au premier service dont la pression est inférieure à VSB1, est déclarée comme vide dans ce premier service, mais si sa pression est supérieure à VSB2, elle peut être transférée dans le deuxième service pour continuer à être utilisée jusqu'à être considérée comme vide dans ce deuxième service.

D'une façon générale, l'invention concerne alors aussi une installation de gestion des bouteilles de gaz mobiles configurée pour mettre en œuvre la méthode selon l'invention, telle que décrite ci-avant, au sein d'un établissement hospitalier comprenant plusieurs services de soins entre lesquels transitent des bouteilles de gaz et dans lesquels lesdites bouteilles de gaz sont utilisées, comprenant :
- des moyens de traitement de données à microprocesseur, tel un serveur,
- une pluralité de récepteurs, agencés au moins dans les services de soins de établissement hospitalier et configurés pour recevoir au moins une information de quantité de gaz transmise par les bouteilles de gaz présentes dans lesdits services de soins et pour transmettre ladite au moins une information de quantité de gaz aux moyens de traitement de données,
- au moins un écran d'affichage, qui est préférentiellement présent dans l'établissement hospitalier,
et dans laquelle :
- les moyens de traitement de données sont configurés pour :
   a) traiter ladite au moins une information de quantité de gaz (Q_{g}) transmise pour chaque bouteille de gaz, et en déduire si chaque bouteille de gaz est une bouteille de gaz pleine, une bouteille de gaz vide ou une bouteille en cours d'utilisation, et
   b) commander sur ledit au moins un écran d'affichage, un affichage pour un ou des services de soins, d'au moins :
      - le nombre de bouteilles de gaz pleines (#P) présentes dans le ou les services de soins considérés et
      - le nombre de bouteilles de gaz vides (#V) présentes dans le ou les services de soins considérés,
      et préférentiellement :
      - le nombre de bouteilles de gaz en cours d'utilisation présentes dans le ou les services de soins considérés et/ou
      - le nombre total de bouteilles de gaz présentes dans le ou les services de soins considérés.

Une telle installation est particulièrement bien adaptée à la gestion des bouteilles d'oxygène mobiles au sein des établissements hospitaliers.

## Revendications

1. Méthode de gestion des bouteilles de gaz (2) mobiles au sein d'un établissement hospitalier (1) comprenant plusieurs services de soins (3a-3d) entre lesquels transitent des bouteilles de gaz (2) et dans lesquels lesdites bouteilles de gaz (2) sont utilisées, dans laquelle :
a. chaque bouteille de gaz (2) est équipée d'un robinet de distribution de gaz (4) comprenant un dispositif électronique (5) configuré pour déterminer et fournir au moins une information de quantité de gaz (Q_{g}) dans la bouteille de gaz (2) considérée, et comprenant en outre un module de communication sans fil configuré pour transférer (T) ladite au moins une information de quantité de gaz (Q_{g}), via un protocole de communication sans fil, et
b. l'établissement hospitalier (1) est équipé d'une pluralité de récepteurs (6), agencés au moins dans les services de soins (3a-3d) et configurés pour recevoir ladite au moins une information de quantité de gaz (Q_{g}) transmise par chaque bouteille de gaz (2) présente dans lesdits services de soins (3a-3d) et pour transmettre (T') ladite au moins une information de quantité de gaz (Q_{g}) à des moyens de traitement de données (7),
**caractérisée en ce que** les moyens de traitement de données (7) sont configurés pour :
i. traiter ladite au moins une information de quantité de gaz (Q_{g}) transmise pour chaque bouteille de gaz (2), et en déduire si chaque bouteille de gaz (2) est une bouteille de gaz pleine ou une bouteille de gaz vide et/ou éventuellement une bouteille en cours d'utilisation, et
ii. commander sur au moins un écran d'affichage (8), un affichage (9) pour un ou des services de soins (3a-3d), d'au moins :
- le nombre de bouteilles de gaz pleines (#P) présentes dans le ou les services de soins (3a-3d) considérés, et
- le nombre de bouteilles de gaz vides (#V) présentes dans le ou les services de soins (3a-3d) considérés,
et préférentiellement :
- le nombre de bouteilles de gaz en cours d'utilisation présentes dans le ou les services de soins (3a-3d) considérés et/ou
- le nombre total de bouteilles de gaz présentes dans le ou les services de soins (3a-3d) considérés.

2. Méthode selon la revendication 1, **caractérisée en ce que** le dispositif électronique (5) est configuré pour déterminer et fournir au moins une information de quantité de gaz (Q_{g}) choisie parmi une pression de gaz ou un volume de gaz.

3. Méthode selon la revendication 1, **caractérisée en ce que** les moyens de traitement de données (7) sont configurés pour associer une bouteille de gaz (2) donnée à un service de soins (3a-3d) donné, lorsque la bouteille de gaz (2) considérée est détectée par un récepteur (6) agencé dans le service de soins (3a-3d) considéré, de préférence pendant une durée (dt) prédéfinie.

4. Méthode selon la revendication 1, **caractérisée en ce que** chaque bouteille de gaz (2) est identifiée par une identification (ID) de bouteille spécifique mémorisée par le dispositif électronique (5) et/ou chaque récepteur (6) est identifié par une identification de récepteur (ID_{R}).

5. Méthode selon la revendication 4, **caractérisée en ce que** le dispositif électronique est configuré pour associer l'information de quantité de gaz relative à une bouteille de gaz donnée (2) à l'identification (ID) de la bouteille de gaz considérée (2) et à l'identification de récepteur (ID_{R}) ayant reçu l'information de quantité de gaz relative à la bouteille de gaz considérée (2).

6. Méthode selon l'une des revendications 4 ou 5, **caractérisée** ce que le module de communication sans fil est configuré pour transférer l'information de quantité de gaz correspondant à chaque bouteille de gaz (2) associée à l'identification (ID) de chaque bouteille de gaz (2) considérée et à l'identification de récepteur (ID_{R}) correspondant.

7. Méthode selon la revendication 1, **caractérisée** ce que les moyens de traitement de données (7) sont configurés pour commander l'affichage (9) sur ledit au moins un écran d'affichage (8), pour au moins un service de soins (3a-3d), des bouteilles de gaz (2) réparties par taille ou contenance et/ou en fonction du gaz qu'elles contiennent, de préférence pour plusieurs services de soins (3a-3d), notamment pour tous les services de soins (3a-3d).

8. Méthode selon la revendication 1, **caractérisée** ce que les moyens de traitement de données (7) sont en outre configurés pour déterminer et commander l'affichage (9) sur ledit au moins un écran d'affichage (8):
- du nombre de bouteilles de gaz (2) pleines à livrer à un premier service de soins (3a-3d), et/ou
- du nombre de bouteilles de gaz (2) dudit premier service de soins (3a-3d) à affecter à un second service de soins (3a-3d),
en fonction du nombre de bouteilles vides et/ou pleines, du nombre total de bouteilles et de la dotation desdits premier et/ou second service de soins (3a-3d).

9. Méthode selon l'une des revendications 1 ou 2, **caractérisée** ce que les moyens de traitement de données (7) sont configurés pour comparer ladite au moins une information de quantité de gaz (Q_{g}) transmise pour chaque bouteille de gaz (2) comprise dans un même service de soin (3a-3d) avec au moins une valeur-seuil haut (VS) et une valeur-seuil bas (VSB) associée au service de soin considéré (3a-3d) et pour en déduire si chaque bouteille de gaz (2) est :
▪ une bouteille de gaz pleine lorsque ladite au moins une information de quantité de gaz (Q_{g}) est supérieure ou égale à ladite valeur-seuil haut (VSH), ou
▪ une bouteille de gaz vide lorsque ladite au moins une information de quantité de gaz (Q_{g}) est inférieure à ladite valeur-seuil bas (VSB), ou
▪ une bouteille de gaz en cours d'utilisation lorsque ladite au moins une information de quantité de gaz (Q_{g}) est comprise entre les valeur-seuil haut (VSH) et valeur-seuil bas (VSB).

10. Méthode selon la revendication 9, **caractérisée en ce que** les valeur-seuil haut (VSH) et valeur-seuil bas (VSB) sont mémorisées par les moyens de traitement de données (7) et/ou sont paramétrables.

11. Méthode selon l'une des revendications 4, 5 ou 6, **caractérisée en ce que** les moyens de traitement de données (7) sont configurés pour retrouver le service de soin considéré (3a-3d) correspondant à l'identification de récepteur (ID_{R}).

12. Méthode selon l'une des revendications précédentes, **caractérisée** ce que les correspondances entre les identifications de récepteur (ID_{R}) et les services de soins sont mémorisée au sein de moyens de mémorisation, notamment au sein des moyens de traitement de données (7).

13. Méthode selon l'une des revendications 9 ou 10, **caractérisée** ce que la valeur-seuil haut (VSH) est supérieure ou égale à 150 bar environ et la valeur-seuil bas (VSB) est inférieure ou égale à 50 bar environ

14. Méthode selon l'une des revendications précédentes, **caractérisée** ce que les bouteilles contiennent un gaz médical choisi parmi l'oxygène ou l'air médicinal ou un mélange NO/N₂, O₂/N₂O, He/O₂, Ar/O₂, Ar/N₂/O₂ ou Xe/O₂.

15. Installation de gestion des bouteilles de gaz (2) mobiles configurée pour mettre en œuvre la méthode selon l'une des revendications précédentes, au sein d'un établissement hospitalier (1) comprenant plusieurs services de soins (3a-3d) entre lesquels transitent des bouteilles de gaz (2) et dans lesquels lesdites bouteilles de gaz (2) sont utilisées, comprenant :
- des moyens de traitement de données (7) à microprocesseur,
- une pluralité de récepteurs (6), agencés au moins dans les services de soins (3a-3d) de établissement hospitalier (1) et configurés pour recevoir au moins une information de quantité de gaz transmise par les bouteilles de gaz (2) présentes dans lesdits services de soins (3a-3d) et pour transmettre (T') ladite au moins une information de quantité de gaz aux moyens de traitement de données (7),
- au moins un écran d'affichage (8),
**caractérisée en ce que** les moyens de traitement de données (7) sont configurés pour :
a) traiter ladite au moins une information de quantité de gaz (Q_{g}) transmise pour chaque bouteille de gaz (2), et en déduire si chaque bouteille de gaz (2) est une bouteille de gaz pleine, une bouteille de gaz vide ou une bouteille en cours d'utilisation, et
b) commander sur ledit au moins un écran d'affichage (8), un affichage (9) pour un ou des services de soins (3a-3d), d'au moins:
- le nombre de bouteilles de gaz pleines (#P) présentes dans le ou les services de soins (3a-3d) considérés et
- le nombre de bouteilles de gaz vides (#V) présentes dans le ou les services de soins (3a-3d) considérés,
et préférentiellement :
- le nombre de bouteilles de gaz en cours d'utilisation présentes dans le ou les services de soins (3a-3d) considérés et/ou
- le nombre total de bouteilles de gaz présentes dans le ou les services de soins (3a-3d) considérés.
